# EUROPEAN PATENT APPLICATION

(11) **EP 4 151 615 A1**
(43) Date of publication of application: **22.03.2023**
(21) Application number: 20742788.1
(22) Date of filing: 11.05.2020
(51) Int. Cl.: C07C 69/587, A61P 35/00, A61K 31/231

(54) **NEW TRIGLYCERIDE OBTAINED FROM A MACROCYBE TITANS EXTRACT, CLINICAL APPLICATIONS AND PRODUCTION METHOD**

(71) Applicant: Bio Tech Stare Global S.r.l., San Jose, 11305 (CR)
(72) Inventor: SEGURA SALAZAR, Fabián, Antonio, SAN JOSE, 11305 (CR)
(74) Representative: Fernandez Pou, Felipe
(86) International application number: PCT/ES2020/070297
(87) International publication number: WO 2021/229109

(57) **Abstract**

The present invention relates to a new triglyceride, of the formula 2R-TG (C18:2, 9z, 12z; C16:0; C18:1, 9z), extracted from an extract of the *Macrocybe titans* fungus. Likewise, its use as a medicine and its different clinical applications in the treatment of diseases regulated by the actin cytoskeleton are contemplated. Its use as an antitumoral agent in cancer diseases is mainly contemplated. Finally, the invention relates to the process for obtaining the *Macrocybe titans* extract that comprises the triglyceride of the invention.

## Description

### Technical field

The present invention belongs to the Biomedicine sector. More specifically, it includes extracts of fungal origin and their clinical applications. In particular, it refers to an extract of *Macrocybes titans* fungus that comprises a triglyceride that acts as a modifier of the biological response, which leads to important clinical applications.

Likewise, the procedure for obtaining said extract is provided.

### Background art

Cancer is a devastating disease with a high incidence. In 2012 there were 14.1 million new cancer diagnoses worldwide and 8.2 million deaths caused by this group of diseases. It is estimated that by 2030 there will be about 23.6 million new cases per year (Cancer Research UK. Worldwide cancer statistics. https://www.cancerresearchukorg/health-professional/cancer-statistics/worldwide-cancer#heading-Zero2018*).*

Apart from the serious cost in human lives, this disease also has a strong impact on the health economy of developed countries. For example, the expense associated with cancer treatment in the United States in 2017 was $ 147.3 billion (NIH. Cancer statistics. https://www cancer gov/about-cancer/understanding/statistics 2018*).*

The fight against cancer has several fronts. Firstly, prevention is revealed as the most effective form of intervention, although it requires a strong political and social commitment to change harmful habits and avoid the main causes of cancer *(*Vineis P, Wild CP. Global cancer patterns: causes and prevention. Lancet 2014; 383:549-57)*.* But the development of new drugs that can contain the disease once detected is also important *(*Magalhaes LG, Ferreira LLG, Andricopulo AD. Recent Advances and Perspectives in Cancer Drug Design. An Acad Bras Cienc 2018; 90:1233-50)*.*

Traditionally, one way of obtaining compounds with therapeutic properties has been the screening of natural products, so that up to 60% of the drugs used today to fight against cancer or infectious diseases have a natural origin *(*Rates SM. Plants as source of drugs. Toxicon 2001; 39:603-13*;* Newman DJ, Cragg GM. Natural products as sources of new drugs over the last 25 years. J Nat Prod 2007;70:461-77*).*

Furthermore, these natural products can become leading compounds, from which families of similar compounds can be generated by means of synthetic chemistry techniques and rational engineering and thus obtaining new functions that were not present in the original compound (Hamburger M, Hostettmann K. Bioactivity in plants: The link between phytochemistry and medicine. Phytochemistry 1991; 30:3864-74)*.*

The therapeutic use of fungi is well known in traditional oriental medicine and these organisms have served as a fertile source of pharmacologically active compounds, providing substances with antimicrobial, antiviral, antitumor, antialergenic, immunomodulatory, anti-inflammatory, etc. capacity *(*Lindequist U, Niedermeyer TH, Julich WD. The pharmacological potential of mushrooms. Evid Based Complement Alternat Med 2005; 2:285-99*;* Strader CR, Pearce CJ, Oberlies NH. Fingolimod (FTY720): a recently approved multiple sclerosis drug based on a fungal secondary metabolite. J Nat Prod 2011; 74:900-7)*.*

Through a collaboration with the National Institute of Biodiversity (INBio) of Costa Rica, the authors of the present invention have studied the antitumoral capacity of various extracts of native fungi from the tropical forests of this country, looking for new natural products with therapeutic application. As a result, they have identified a new compound capable of stopping tumor growth in vivo from the *Macrocybe titans* fungus.

The *Macrocybe* genus groups seven species distributed in tropical and subtropical areas of the world that produce large carpophores, reaching up to one meter in diameter and 18 Kg in weight *(*Pegler DN, Lodge Dj, Nakasone KK. The pantropical genus Macrocybe gen. nov. Mycologia 1998; 90:494-504)*.* The *Macrocybe titans* species is the only one of this genus found in the American continent (Ramirez NA, Niveiro N, Michlig A, Popoff OF. First record of Macrocybe titans (Tricholomataceae, Basidiomycota) in Argentina. Check List 2017; 13:1538*).* Although they are edible species *(*Razaq A, Nawaz R, Khalid AN. An Asian edible mushroom, Macrocybe gigantea: its distribution and ITS-rDNA based phylogeny. Mycosphere 2016; 7:525-30)*,* their use as therapeutic fungi is not described in the literature. It has only been suggested that some *Macrocybe titans* polysaccharides may inhibit melanoma cell migration *(*Milhorini SDS, Smiderle FR, Biscaia SMP, Rosado FR, Trindade ES, lacomini M. Fucogalactan from the giant mushroom Macrocybe titans inhibits melanoma cells migration. Carbohydr Polym 2018; 190:50-6) and that the productive body of *Macrocybe gigantea* contains antimicrobial compounds (Gaur T, Rao PB. Analysis of Antibacterial Activity and Bioactive Compounds of the Giant Mushroom, Macrocybe gigantea (Agaricomycetes), from India. Int J Med Mushrooms 2017; 19:1083-92*).*

### Brief description of figures

**Figure 1****.** Toxicity analysis in lines A549 (tumor) and NL20 (non-tumor) with the initial extract of M. *titans.* IC50 for the tumor line <0.1 mg / ml; IC50 for the non-tumor line = 0.5 mg / ml.
**Figure 2****.** 1H- NMR spectrum of the purified extract. The typical protons of glycerol, those of alkyl and olefin groups, are indicated.
**Figure 3****.** 13C-NMR spectrum of the purified extract. The typical carbons of glycerol, those of alkyl, olefin and carbonyl groups are indicated.
**Figure 4****.** A549 and NL20 cells stained with fluorescent falacidine that marks actin fibers (in green), before (Control) and after being treated (Treated) with TG10.
**Figure 5****.** Evolution of tumor volume in the xenograft experiment. Tumors injected with vehicle (Control, diamonds) or with TG10 (TG, squares). The mean of the volumes for each group over time is represented (d = days from the injection of the tumor cells).

### Detailed description of the invention

The authors of the present invention have identified from a *Macrocybe titans* fungus extract a new compound capable of stopping tumor growth in vivo.

A main aspect of the invention provides said compound, which is a triglyceride of formula 2R TG (C18: 2, 9z, 12z; C16: 0; C18: 1, 9z), hereinafter TG10, represented by the following scheme:

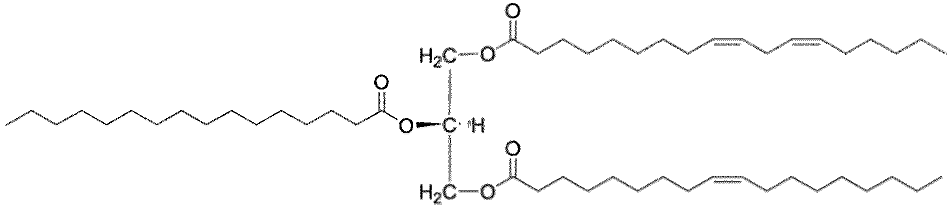

This compound acts as a biological response modifier (BRM) since it specifically alters the actin cytoskeleton. Therefore, it has direct applications in all those phenomena regulated by this component of the cytoskeleton, including in a non-exclusive way: i) changes in cell morphology, ii) cell motility (including metastasis), iii) muscle contraction, iv) modification of cell-cell junctions, v) regulation of endocytosis and phagocytosis, vi) embryogenesis, vii) control of gene expression, viii) regulation of the immune system, ix) defense against pathogens (bacteria, fungi, viruses, parasites, etc. ) and x) regulation of apoptosis, among others.

Due to its properties, in a second aspect of the invention, the TG10 triglyceride of the invention is provided for use as a medicine.

The TG10 triglyceride positively influences all those diseases related to the actin cytoskeleton. Thus, another aspect of the invention provides the use of triglyceride in the treatment of actin cytoskeleton regulated diseases such as, but not limited to, amyloidosis, retinitis pigmentosa, infectious diseases, kidney diseases and congenital deafness syndrome.

The triglyceride of the invention has been shown to be effective in reducing the size of human tumors. Thus, another main aspect of the invention provides the use of TG10 triglyceride in the treatment of cancer diseases, both in animals and in human patients, by itself or in combination with other therapies (radiotherapy, chemotherapy, immunotherapy, etc).

Furthermore, the authors of the present invention have shown that triglyceride binds preferentially to tumor cells, therefore, together with a tracer (fluorescent, radioactive, etc.), it allows locating tumors in a living organism, both in an animal as in a human patient, in the context of diagnostic imaging devices (PET, fluorescence, etc.). Said affinity of the triglyceride for tumor cells also allows drugs or nanoparticles to be directed towards tumor cells, as therapeutic treatments.

In another main aspect of the invention a pharmaceutical composition comprising the triglyceride of the invention is provided.

As mentioned above, the authors of the present invention obtained triglyceride from an extract of *Macrocybe titans.* Thus, another main aspect of the invention provides the procedure for obtaining an extract of *Macrocybe titans* that comprises the following steps:
a. subjecting a sample of a *Macrocybe titans* fungus to an extraction in 95% ethanol,
b. treating the extract obtained in a) with ultrasound for 30-60 minutes, preferably 30 min,
c. maintaining the extract treated in b) at a temperature between -20 and -80°C, preferably -20°C, for 24-48 hours, preferably 24 hours,
d. obtaining the precipitated phase of the extract thus treated, and
e. from the precipitated phase of the extract obtained in d), carrying out an alkaloid separation followed by a column chromatography with polymeric resin using an 8:2 IP:CH2L2 mobile phase at pH 12, leading to a fraction of the extract that comprises the triglyceride of formula 2R-5 TG (C18:2, 9z,12z; C16:0; C18:1, 9z).

Once the triglyceride of the invention has been identified, and its formula determined, it is also possible to obtain it by means of its chemical synthesis, instead of extracting it from the original source, which has advantages from an economic, reproducibility and pharmacological safety point of view.

### Examples

### Examples 1. Iterative screening by toxicity analysis and separation of new fractions by chromatography.

Extracts (aqueous, ethyl, acidic) were obtained from 9 macrofungi species collected by INBio in the Costa Rican rainforests. All of them were subjected to a cytotoxicity test with two human cell lines: A549 (lung adenocarcinoma) and NL20 (immortalized, non-tumor line, obtained from the pulmonary epithelium). Cells were plated in 96-well plates at densities of 2,000 (A549) or 10,000 (NL20) cells per well. Once the cells were stuck to the bottom, serial concentrations of each extract were added and incubated at 37 ° C for 5 days under a humidified atmosphere (85% relative humidity) and containing 5% CO2. Eight repeats (wells) were studied for each concentration. At the end of the incubation process, 20 µl of Cell Titer (Promega) were added per well and left for 4 h to allow the formation of the colored formazan salts. Colorimetric quantification was performed with a plate reader (POLARstar Omega) using a wavelength of 490 nm, following already published protocols *(*Coderch C, Diaz de CM, Zapico JM, Pelaez R, Larrayoz IM, Ramos A, et al. In silico identification and in vivo characterization of small molecule therapeutic hypothermia mimetics. Bioorg Med Chem 2017;25:6597-604*).* The selection criteria for the extracts consisted of choosing those with toxicity to tumor cells even at low concentrations while respecting the integrity of non-tumor cells, or at least providing a wide therapeutic window between both cell lines. The extracts selected in the first round were fractionated with different columns and chromatographic protocols. Each new peak was separated and subjected to toxicity analysis again. This process was repeated as many times as necessary to finally obtain a sufficiently pure fraction whose majority compound could be identified by analytical chemistry methods.

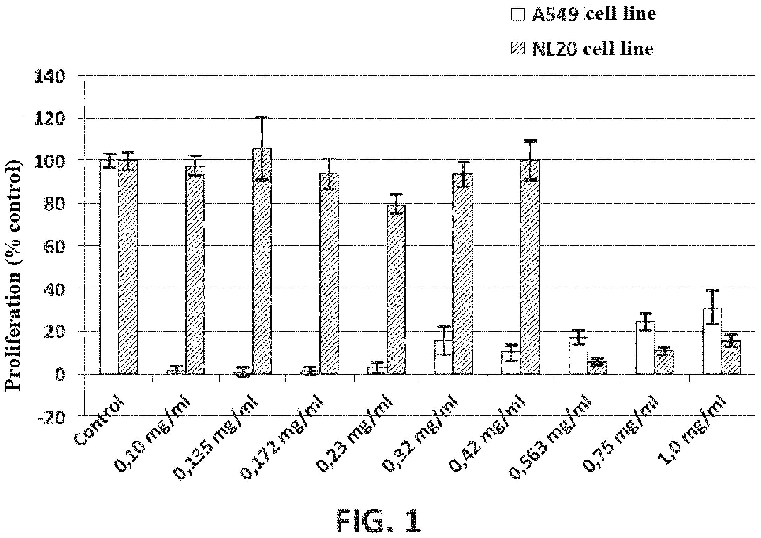

Results: Among all the extracts analyzed in the first round, the one corresponding to the M. *titans* species was the one with the widest therapeutic window since the A549 cells were destroyed at all concentrations, even at 0.1 mg / ml, while the NL20 cells survived without problems even at concentrations of 0.42 mg / ml (Figure 1). Among the different extracts of this species, the most effective was that obtained by extraction in 95% ethanol, treated with ultrasound for 30 min, kept at -20°C for 24h and, specifically, the precipitated phase after this treatment. Due to the lipid nature of this extract, it was decided to perform an alkaloid separation followed by column chromatography with polymer resin (HP20-SS) using different mobile phases. All the fractions obtained were subjected to toxicity tests again. The fraction that maintained the best therapeutic window corresponded to that obtained at pH 12 and an 8: 2 IPA:CH2CI2 mobile phase.

### Example 2. Chemical characterization of the molecule responsible for antitumor

### activity.

The fraction obtained in the previous experiment was used to elucidate the chemical structure of the antitumor product. For this, nuclear magnetic resonance (NMR) analyzes with 1H, 13C and DEPT-135, mass spectroscopy (Q-TOF) and the twodimensional HMQC and COSY experiments were performed.

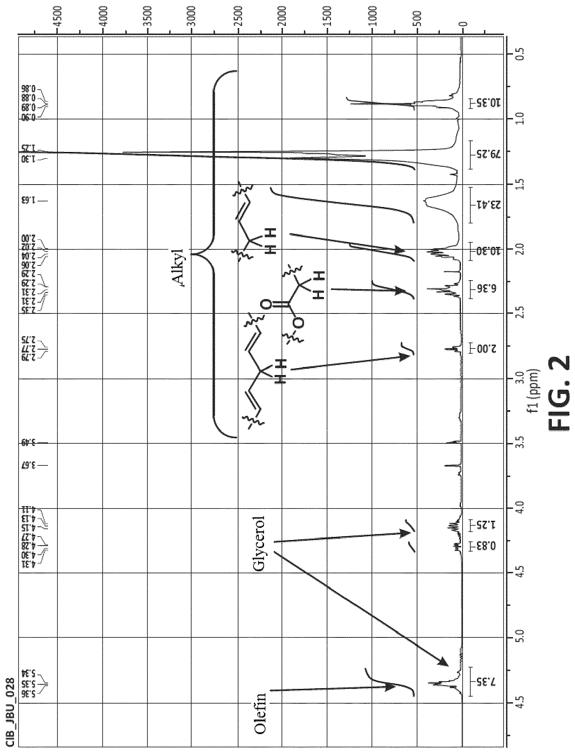

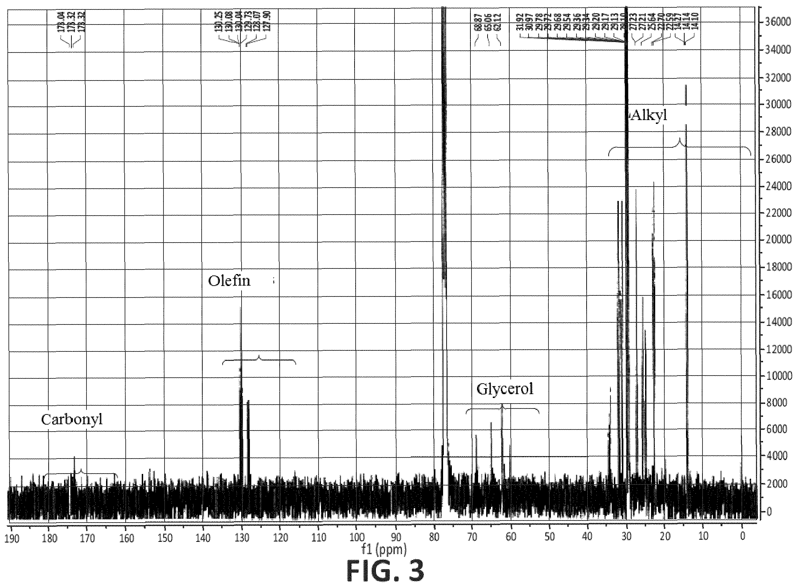

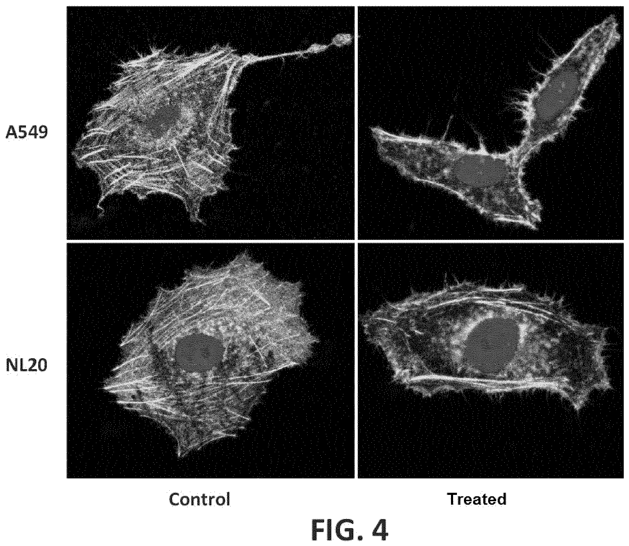

Results: The 1H-NMR study indicated that the product contained many alkyl protons, a series of α-protons to alcohols, and several olefin protons. The distribution of the protons indicated that the natural extract contained a triglyceride (TG) mixed with several of its component fatty acids. A triplet at 2.77 ppm recognized two protons of a methylene located between two double bonds, indicating a double unsaturation in one of the fatty acids (Figure 2). The 13C and DEPT-135 experiments allowed assigning the product carbons (Figure 3). Two dimensional experiments confirmed the assignment of carbons and protons in the TG. On the other hand, the exact mass determination (MS-QTOF) allowed identifying the fatty acids that made up the TG. All this led to suggest that the TG contained three different fatty acids: oleic (C18: 1, 9z), palmitic (C16: 0), and a doubly unsaturated fatty acid of 18 or 20 carbons. However, since the sample was not pure and it was difficult to accurately assign the order and size of the fatty acids, it was decided to synthesize a series of similar TGs to empirically determine the antitumor activity of each one. For some TGs that had optical activity, the two enantiomers were synthesized (Table 1). Toxicity tests indicated that of all TGs synthesized, only TG10 [2R-TG (C18: 2, 9z, 12z; C16: 0; C18: 1, 9z)] (Figure 4) exhibited antitumor activity against A549 and it was not toxic to normal cells (NL20). The other TGs had no appreciable activity on the cells. It should be noted that the S enantiomer (TG9) of active TG (TG10) also had no effect on cells, indicating a high degree of specificity for this activity.

**Table 1. Triglycerides (TG) synthesized to check its antitumor activity**

| Internal code | Formula |
|---|---|
| TG1 | TG (C16:0; C18:1, 9z; C20:2, 11z,14z) |
| TG2 | TG (C16:0; C20:2, 11z,14z; C18:1, 9z) |
| TG3 | TG (C16:0; C18:1, 9z; C18:1, 9z) |
| TG4 | TG (C18:2, 9z,12z; C18:0; C18:1, 9z) |
| TG5 | 2S-TG (C20:2, 11z,14z; C18:1, 9z; C16:0) |
| TG6 | 2R-TG (C20:2, 11z,14z; C18:1, 9z; C16:0) |
| TG7 | 2S-TG (C20:2, 11z,14z; C16:0; C18:1, 9z) |
| TG8 | 2R-TG (C20:2, 11z,14z; C16:0; C18:1, 9z) |
| TG9 | 2S-TG (C18:2, 9z,12z; C16:0; C18:1, 9z) |
| TG10 (active) | 2R-TG (C18:2, 9z,12z; C16:0; C18:1, 9z) |

### Example 3. In vitro tests: modulation of the actin cytoskeleton.

Many antitumor substances exert their effects by modulating the cytoskeleton of tumor cells, either acting on microtubules or on actin filaments *(*Zhang S, Menche D, Zahler S, Vollmar AM, Liebl J, Forster F. In vitro anti-cancer effects of the actin-binding natural compound rhizopodin. Pharmazie 2015;70:610-5*.).* To check whether compound TG10 has a similar mechanism of action as other antitumor drugs, tumor cells (A549) and non-tumor cells (NL20) were subjected to a 24-hour treatment with 0.03 mg / ml TG10. At the end of this time, the cells were fixed with 10% formaldehyde for 10 min, permeabilized with 0.1% Triton X-100 for 10 min and exposed to 1: 200 Bodipy-falacidine and 1: 1000 DAPI (Molecular Probes) for 1 h. The images were obtained with a confocal laser microscope (TCS SP5, Leica).

Results: As expected, both A549 and NL20 cells have typical stress fibers (composed of actin filaments) in their cytoplasm before treatment. After TG10 treatment, A549 cells completely lost the polymerized actin fibers and this passed to the cell periphery, where small filopodia were formed. In contrast, NL20 (non-tumor) cells maintained the filamentous structure of actin even after TG10 treatment (Figure 4). These results confirmed that TG10 acts on the actin cytoskeleton of tumor cells.

### Example 4. In Vivo Assays: Human Lung Cancer Xenografts in Immunosuppressed

### Mice.

The usual method of demonstrating that a new antitumor drug works in vivo is to perform a xenograft experiment using human tumor cells injected into immunodeficient mice (Zitvogel L, Pitt JM, Daillere R, Smyth MJ, Kroemer G. Mouse models in oncoimmunology. Nat Rev Cancer 2016;16:759-73*).* In this case, 20 male mice of the NOD scid gamma strain (NSG, Stock No. 005557, The Jackson Laboratory) were used. All of them were injected subcutaneously with 10 million A549 cells and left for 2 weeks until the tumors became measurable. Two groups of 10 mice each were randomized and marked as control group (diamonds) and TG10-treated group (squares). From this point, intratumor injections with 100 µl of PBS (control group) or 0.1 mg / ml of TG10 in PBS (treated group) were applied 3 times per week.

Before each injection, tumor volumes were measured with a caliper. The mice were euthanized when the tumors reached a size incompatible with animal welfare.

Results: TG10 injections started on day 15 after the initial injection of tumor cells.

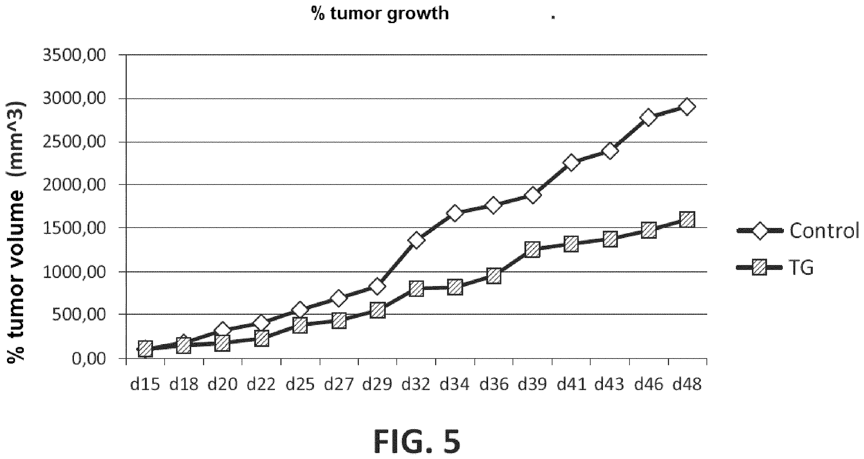

From this point on, the size of the tumors injected with PBS continued to grow gradually until reaching the maximum size allowed (for reasons of ethics and animal welfare) on day 48. On the other hand, the tumors injected with TG10 continued to grow, but at a slower rate than that of the tumors of the control group (Figure 5). At the end of the experiment, the tumors of the control group had an average volume of 2900 mm3, while those treated had an average volume of 1600 mm3. A two-way analysis of variance (ANOVA) was performed which showed that there is a significant difference between the volume of the treated tumors and the control ones (p <0.05) due to the treatment.

## Claims

1. Triglyceride of formula 2R-TG (C18:2, 9z, 12z; C16:0; C18:1, 9z).

2. Triglyceride according to claim 1, for its use as a medicine.

3. Triglyceride according to claim 2 for its use in the treatment of diseases regulated by the actin cytoskeleton.

4. Triglyceride according to claim 3, wherein the disease regulated by the actin cytoskeleton is selected from amyloidosis, retinitis pigmentosa, infectious diseases, kidney diseases and congenital deafness syndrome.

5. Triglyceride according to claim 2, for its use in the treatment of cancer diseases.

6. Triglyceride according to claim 1 for its use in the diagnosis of tumors using imaging techniques.

7. Triglyceride according to claim 6 for its use in combination with a tracer.

8. Pharmaceutical composition comprising the triglyceride according to claim 1.

9. *Macrocybe titans* extract comprising the triglyceride of claim 1.

10. Procedure for obtaining an extract of *Macrocybe titans* according to claim 9 comprising the following steps:
a. subjecting a sample of a *Macrocybe titans* fungus to an extraction in 95% ethanol,
b. treating the extract obtained in a) with ultrasound for 30-60 minutes,
c. maintaining the extract treated in b) at a temperature between -20 and -80°C, for 24-48 hours,
d. obtaining the precipitated phase of the extract thus treated, and
e. from the precipitated phase of the extract obtained in d), carrying out an alkaloid separation followed by a column chromatography with polymeric resin using an 8:2 IP:CH2L2 mobile phase at pH 12, leading to a fraction of the extract that comprises the triglyceride of claim 1.
